# EUROPEAN PATENT SPECIFICATION

(11) **EP 3 326 619 B1**
(45) Date of publication and mention of the grant of the patent: **05.01.2022**
(21) Application number: 17203972.9
(22) Date of filing: 28.11.2017
(51) Int. Cl.: A61K 9/20, A61K 31/13, A61K 31/675, A61K 9/28, A61K 31/683, A61K 31/7068

(54) **SOLID ORAL PHARMACEUTICAL COMPOSITIONS COMPRISING TENOFOVIR AND EMTRICITABINE**
FESTE ORALE PHARMAZEUTISCHE ZUSAMMENSETZUNGEN ENTHALTEND TENOFOVIR UND EMTRICITABINE
COMPOSITIONS PHARMACEUTIQUES ORALES SOLIDES COMPRENANT DU TENOFOVIR ET DU EMTRICITABINE

(30) Priority: 29.11.2016 TR 201617448
(43) Date of publication of application: 30.05.2018
(73) Proprietor: SANOVEL ILAC SANAYI VE TICARET A.S., Istanbul 34460 (TR)
(72) Inventor: TÜRKYILMAZ, Ali, 34460 Istanbul (TR); YILDIRIM, Ediz, 34460 Istanbul (TR); DEMIR, Bülent, 34460 Istanbul (TR); MURATOGLU, Yesim, 34460 Istanbul (TR); NURIOGLU, Ayda, 34460 Istanbul (TR); BAS, Oguz, 34460 Istanbul (TR)
(74) Representative: Sevinç, Erkan

(56) References cited:
- WO-A1-2004/064845
- WO-A1-2012/068535
- WO-A2-2008/096369
- WO-A2-2010/059038
- US-A1- 2014 212 491
- US-A1- 2015 141 376
- US-A1- 2016 199 396
- EUROPEAN MEDICINES AGENCY: "Scientific discussion (Truvada)", INTERNET CITATION, 1 February 2005 (2005-02-01), XP002417805, Retrieved from the Internet: URL:http://www.emea.eu.int/humandocs/PDFs/ EPAR/truvada/2832505en6.pdf [retrieved on 2007-01-31]

## Description

### Field of Invention

The present invention relates to pharmaceutical compositions comprising tenofovir or a pharmaceutically acceptable salt thereof and emtricitabine or a pharmaceutically acceptable salt thereof. The invention further discloses a process for preparing tablet formulations thereof.

### Background of Invention

Tenofovir is a nucleotide analogue reverse transcriptase inhibitor. The chemical name of tenofovir is ({[(2R)-1-(6-amino-9H-purin-9-yl)propan-2-yl]oxy}methyl)phosphonic acid and has the structure shown in the following formula I.

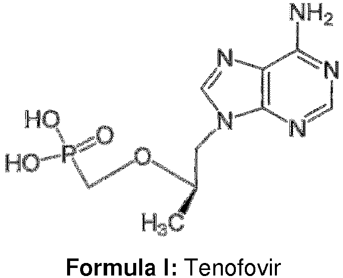

Tenofovir disoproxil fumarate is a prodrug of tenofovir and is marketed under the brand name VIREAD^{®}. Tenofovir disoproxil fumarate blocks the reverse transcriptase enzyme which is crucial for the human immunodeficiency virus 1 (HIV-1) and the hepatitis B virus. Therefore, it is used in the treatment of HIV-1 and hepatitis B. Tenofovir disoproxil fumarate has the following structural formula.

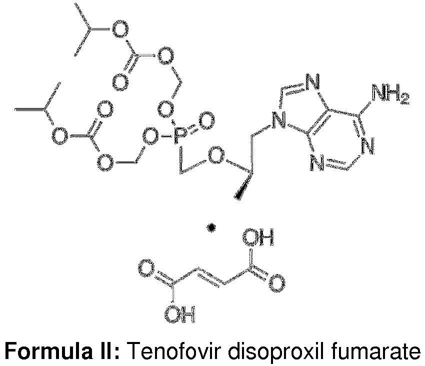

Emtricitabine is a synthetic fluoro derivative of thiacytidine with potent antiviral activity. The chemical name of emtricitabine is 4-amino-5-fluoro-1-[(2R,5S)-2-(hydroxymethyl)-1,3-oxathiolan-5-yl]-1,2-dihydropyrimidin-2-one and has the structure shown in the following formula III.

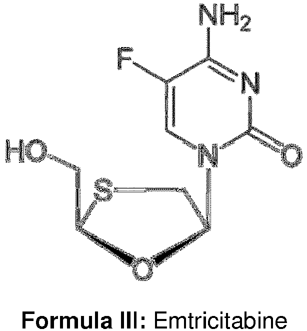

Emtricitabine is phosphorylated to form emtricitabine 5'-triphosphate within the cell. This metabolite inhibits the activity of human immunodeficiency virus (HIV) reverse transcriptase both by competing with the natural substrate deoxycytidine 5'-triphosphate and by incorporation into viral DNA causing a termination of DNA chain elongation.

Emtricitabine, with trade name EMTRIVA^{®}, is a nucleoside reverse transcriptase inhibitor (NRTI) for the treatment of HIV infection in adults and children.

Emtricitabine is also marketed in a fixed-dose combination with tenofovir under the brand name TRUVADA^{®} and it is used for a Pre-Exposure Prophylaxis indication must only be prescribed to individuals confirmed to be HIV-negative immediately prior to initial use and periodically during use.

Several attempts have been made to formulate combination regimens. Combination therapy reduces the daily dosages to be taken by patients and simplifies dosing schedule thereby increases patient compliance. Combination therapy also increases the drug efficacy. Use of combination therapy can yield an equivalent antiviral effect with reduced toxicity.

In the state of the art, there are various combinations of nucleoside reverse transcriptase inhibitors and nucleotide reverse transcriptase inhibitors for the treatment of patients infected with HIV virus. Emtricitabine and penciclovir, emtricitabine and famciclovir, emtricitabine and adefovir, lamivudine and adefovir are some examples to these combinations.

Tenofovir disoproxil fumarate and emtricitabine is also a combination appearing in the state of the art, which exhibits synergistic antiviral activity on HIV virus.

A tablet combination of tenofovir disoproxil fumarate and emtricitabine is disclosed in the prior art wherein pregelatinized starch is used as binder and lactose monohydrate is used as diluent.

In the state of the art, pregelatinized starch and lactose monohydrate are known for their tablet quality enhancing properties. However, in the literature, it is stated that the coexistence of these two excipients in a tablet formulation has some disadvantages such as poor flow, segregation, insufficient disintegration ability and dissolubility problems. It is also mentioned that pregelatinized starch causes tablet deformation and tendency to generate dust due to this coexistence.

The patent application no. WO2007068934 A2 in the prior art reveals a dosage form combination of a nucleoside reverse transcriptase inhibitor and a nucleotide reverse transcriptase inhibitor. This dosage form comprises at least two layers in which these two inhibitor groups are present separately since they are incompatible. The preferred embodiment of the dosage form asserts 3 layered form which comprises an intermediate layer free of active ingredient. This dosage form is intended to comprise binders such as starch derivatives, HPMC, HPC or povidone in the amount of 1-50% by weight of the total composition.

Further another patent application no. WO2015085976 A1 discloses a pharmaceutical combination of tenofovir disoproxil fumarate and another active substance such as emtricitabine. The essential feature of this combination is the existence of a disintegrant selected from the group including crospovidone, maize starch, and hydroxypropylcellulose. Additionally, it is stated that the combination comprises an acid to ensure the stability. WO2012068535 A1 discloses multilayer tablets that contain rilpivirine hydrochloride, emtricitabine, and tenofivir disoproxil fumarate. The exemplified formulations contain starch.

Thus, there is still a need for a pharmaceutical composition for the treatment of HIV-1 infection which will eliminate the interaction risk due to the incompatibility of active ingredients and which will assure stability, therapeutic safety, efficacy and high bioavailability during the shelf life accordingly.

Moreover, there is no disclosure or teaching/suggestion in the prior art about how to develop a stable combination of tenofovir disoproxil fumarate and emtricitabine which is substantially free of starch and which can also exhibit rapid disintegration as well as equivalent in vitro dissolution profile.

### Objects and Brief Description of the Invention

The main object of the present invention is to obtain combination formulations comprising tenofovir disoproxil fumarate (tenofovir DF) and emtricitabine eliminating all aforesaid problems and bringing additional advantages to the relevant prior art. The scope of the invention is defined by the appended claims.

Another object of the present invention is to obtain combination formulations of tenofovir DF and emtricitabine with high stability and bioavalibility.

A further object of the present invention is to develop combination formulations of tenofovir DF and emtricitabine having an improved level of dissolution rate and solubility.

The inventive tablet formulation of tenofovir DF and emtricitabine is substantially free of starch.

Yet, another object of the present invention is to provide a tenofovir DF and emtricitabine tablet comprising at least one film coating to protect the pharmaceutical composition against the moisture to maintain the stability.

A further object of the present invention is to improve a process for preparing the said coated tablet comprising wet granulation and coating.

### Detailed Description of Invention

In accordance with the objects outlined above, detailed features of the present invention are given herein.

The present invention relates to pharmaceutical compositions comprising tenofovir or a pharmaceutically acceptable salt thereof and emtricitabine or a pharmaceutically acceptable salt thereof as active agents, wherein the composition is substantially free of starch.

According to the preferred embodiment of the invention, the active agents are tenofovir disoproxil fumarate (tenofovir DF) and emtricitabine.

According to this preferred embodiment, the amount of tenofovir DF is between 5-60% by weight of the total composition. Preferably this amount is between 10-50% by weight of the total composition. More preferably tenofovir DF is present between 20-40% by weight in the total composition.

According to this preferred embodiment, the amount of emtricitabine is between 5-60% by weight of the total composition. Preferably this amount is between 5-40% by weight of the total composition. More preferably emtricitabine is present between 10-30% by weight in the total composition.

Tenofovir DF is present in an amount of 10 to 600 mg, preferably 100 to 500 mg and more preferably 200 to 400 mg. 300 mg of tenofovir DF is equivalent to 245 mg of tenofovir.

On the other hand, emtricitabine is present in an amount of 10 to 600 mg, preferably 50 to 400 mg and more preferably 100 to 300 mg.

In the preferred embodiment of the invention, the ratio of tenofovir DF to emtricitabine is in the range of 3:1 to 0.5:1 and preferably 2:1 to 1:1. In the most preferred embodiment, this ratio is 1.5:1.

According to these embodiments, the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet, orally administrable film.

The composition is preferably in the form of a film-coated tablet.

According to the preferred embodiment of the invention, the composition is essentially free of binder. According to this embodiment, the composition comprises at least one pharmaceutically acceptable excipient selected from diluents, disintegrants, lubricants, or mixtures thereof.

According to one embodiment of the invention, the solid oral pharmaceutical composition comprises at least one diluent which is selected from the group comprising lactose monohydrate, lactose, dibasic calcium phosphate, microcrystalline cellulose, mannitol, spray-dried mannitol, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to the preferred embodiment of the invention, the solid oral pharmaceutical composition comprises two diluents which are lactose monohydrate and microcrystalline cellulose.

The amount of lactose monohydrate is 10-15% by weight of the total composition.

The amount of microcrystalline cellulose is between 1-50%, preferably 5-30% and more preferably 15-25% by weight of the total composition.

In the preferred embodiment of the invention, the ratio of microcrystalline cellulose to lactose monohydrate is in the range of 1:1 to 3:1, preferably 1:1 to 2:1 and more preferably 1.5:1 to 2:1 by weight. This preferred selection of range assures the enhanced stability and bioavailability during the shelf life.

According to one embodiment of the invention, the solid oral pharmaceutical composition comprises at least one disintegrant which is selected from the group comprising croscarmellose sodium, sodium carbonate, hydroxylpropyl cellulose (HPC), cross-linked polyvinylpyrrolidone (crospovidon), copovidon, polycarbophil, low-substitue poloxamer, alginic acid and alginates, ion-exchange resins, magnesium aluminum silica, sodium dodecyl sulphate, sodium carboxy methyl cellulose, carboxy methyl cellulose calcium, docusate sodium, guar gum, polyacrylin potasium, sodium alginate, sodium glysin carbonate, sodium lauryl sulphate or mixtures thereof.

According to the preferred embodiment of the invention, the solid oral pharmaceutical composition comprises one disintegrant which is croscarmellose sodium. The amount of croscarmellose sodium is between 5-30%, preferably 10-20% by weight of the total composition.

In the preferred embodiment of the invention, the ratio of croscarmellose sodium to the total weight of diluent is in the range of 1:1 to 1:4, preferably 1:2 to 1:3 and more preferably 1:2 to 1:2.5. This preferred selection of range assures the improved dissolution and disintegration profiles.

According to one embodiment of the invention, the solid oral pharmaceutical composition comprises at least one lubricant and one glidant which are selected from the group comprising sodium lauryl sulphate, sodium stearyl fumarate, magnesium stearate, colloidal silicon dioxide, zinc stearate, calcium stearate, mineral oil, talc, polyethylene glycol, glyceryl monostearate, glyceryl palmitostearate, magnesium lauryl sulphate, fumaric acid, zinc stearate, stearic acid, hydrogenated natural oils, silica, paraffin or mixtures thereof.

According to the preferred embodiment of the invention, the solid oral pharmaceutical composition comprises a lubricant which is magnesium stearate.

The amount of magnesium stearate is between 0.1-5%, preferably 1-2% by weight of the total composition.

According to the preferred embodiment of the invention, the solid oral pharmaceutical composition comprises a glidant which is colloidal silicon dioxide (aerosil).

The amount of colloidal silicon dioxide is between 0.1-3%, preferably 0.2-1% by weight of the total composition. The coexistence of magnesium stearate and colloidal silicon dioxide in the tablet formulation enhances the wettability and disintegration over the prior art.

According to one embodiment of the invention, the solid oral pharmaceutical composition comprises at least one coating layer to protect the composition against the moisture and maintain the stability. Suitable coating ingredients are selected from the group comprising hydroxypropylmethyl cellulose (hypromellose), lactose monohydrate, hydroxypropyl cellulose, polyvinyl alcohol (PVA), polyethylene glycol (PEG), talc, polyvinyl alcoholpolyethylene glycol copolymers (Kollicoat IR), ethylcellulose dispersions (Surelease), polyvinylprolidone, polyvinylprolidone-vinyl acetate copolymer (PVP-VA) and all kinds of OpadryTM, pigments, dyes, titanium dioxide, iron oxide or polymethylmetacrylate copolymers and mixtures thereof.

According to one embodiment, coating layer is Opadry II Blue which comprises lactose monohydrate, hydroxypropylmethyl cellulose (hypromellose), titanium dioxide, polyethylene glycol (PEG), blue pigment (FD&C blue no.2).

According to one preferred embodiment, the solid oral pharmaceutical composition is in the form of coated tablet comprising tenofovir DF and emtricitabine as active agents, lactose monohydrate and microcrystalline cellulose as diluents, croscarmellose sodium as disintegrant, magnesium stearate as lubricant, colloidal silicon dioxide as glidant and a coating layer.

According to this preferred embodiment, the composition comprises;
- 5-60% by weight tenofovir disoproxil fumarate,
- 5-60% by weight of emtricitabine,
- 10-15% by weight of lactose monohydrate,
- 1-50% by weight of microcrystalline cellulose,
- 5-30% by weight of croscarmellose sodium,
- 0.1-5% by weight of magnesium stearate,
- 0.1-3% by weight of colloidal silicon dioxide,
- 1-5% by weight of coating.

These analytically selected ratios ensure the required effective doses for the treatment and enhance the stability and dissolution profile of the film-coated tablet subjected to the invention.

According to all these embodiments, the below given formulations can be used in the solid oral pharmaceutical composition subjected to the invention. These examples are not limiting the scope of the present invention and should be considered under the light of the foregoing detailed disclosure.

### Example 1: Film-coated tablet formulation

| **Ingredients** | **Amount (%)** |
|---|---|
| Tenofovir DF | 20 - 40 |
| Emtricitabine | 10-30 |
| Lactose monohydrate | 10-15 |
| Microcrystalline cellulose (PH101) | 15-25 |
| Croscarmellose sodium | 10 - 20 |
| Magnesium stearate | 1 - 2 |
| Colloidal silicon dioxide | 0.2 - 1 |
| Coating | 1 - 5 |

| **Coating Material (Opadry II Blue) Ingredients** | **Amount (%)** |
|---|---|
| Hypromellose (Methocel E5 LV) | 20 - 40 |
| Titanium dioxide | 20 - 30 |
| Lactose monohydrate | 30 - 50 |
| Polyethylene glycol powder (PEG 4000) | 5 - 15 |
| Blue pigment | 1 - 5 |

### Example 2: Film-coated tablet formulation

| **Ingredients** | **Amount (%)** |
|---|---|
| Tenofovir DF | 30 |
| Emtricitabine | 20 |
| Lactose monohydrate | 12.5 |
| Microcrystalline cellulose | 20.5 |
| Croscarmellose sodium | 15 |
| Magnesium stearate | 1.5 |
| Colloidal silicon dioxide | 0.5 |
| **Total tablet** | **100** |
| Coating | 3 |
| **Coated tablet** | **103** |

The preparation method of the above mentioned pharmaceutical formulations essentially comprises 4 main stages. During the first stage, basic granule structure which can also be called as internal phase is formed by wet granulation. These basic granules comprise the active ingredients and some of the excipients. In the second stage, these granules are processed with disintegrant, lubricant and glidant to form an external phase on them and ameliorate the final tablet product properties such as stability, wettability and disintegration. Third stage includes compressing these granules into tablets. In the fourth stage, a coating layer is performed on these tablets against the moisture to maintain the stability.

The pharmaceutical formulations subjected to the invention are prepared in detail by following these steps:
- mixing tenofovir disoproxil fumarate, emtricitabine, microcrystalline cellulose, lactose monohydrate and one-third by weight of croscarmellose sodium together to prepare a powder mixture
- granulating the powder mixture with water
- drying the granules preferably until they have 1% moisture ratio
- sieving the dried granules through a sieving mesh
- sieving colloidal silicon dioxide and two-thirds by weight of croscarmellose sodium through the same mesh, adding them into the sieved granules and mixing
- adding magnesium stearate and mixing the total mixture
- compressing the total mixture into tablets
- preparing an aqueous solution of coating material and coating the tablets with this solution in a way to form a film layer

## Claims

1. A solid oral pharmaceutical composition comprising tenofovir or a pharmaceutically acceptable salt thereof, emtricitabine or a pharmaceutically acceptable salt thereof and lactose monohydrate and microcrystalline cellulose as diluents, wherein lactose monohydrate is in an amount 10-15% by weight of the composition and the composition is substantially free of starch.

2. The solid oral pharmaceutical composition according to claim 1, wherein the composition comprises tenofovir disoproxil fumarate and emtricitabine.

3. The solid oral pharmaceutical composition according to claim 2, wherein the composition comprises tenofovir disoproxil fumarate in an amount of 5-60%, preferably 10-50% and more preferably 20-40% by weight.

4. The solid oral pharmaceutical composition according to claim 3, wherein the composition comprises emtricitabine in an amount of 5-60%, preferably 5-40% and more preferably 10-30% by weight.

5. The solid oral pharmaceutical composition according to claim 4, wherein the ratio of tenofovir disoproxil fumarate to emtricitabine is in the range of 3:1 to 0.5:1, preferably 2:1 to 1:1 and more preferably this ratio is 1.5:1.

6. The solid oral pharmaceutical composition according to any preceding claims, wherein the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet, orally administrable film.

7. The solid oral pharmaceutical composition according to claim 6, wherein the composition is in the form of a film-coated tablet.

8. The solid oral pharmaceutical composition according to any preceding claims, wherein the composition further comprises at least one pharmaceutically acceptable excipient selected from disintegrants, lubricants, glidants or mixtures thereof.

9. The solid oral pharmaceutical composition according to claim 1, wherein the ratio of microcrystalline cellulose to lactose monohydrate is in the range of 1:1 to 3:1, preferably 1:1 to 2:1 and more preferably 1.5:1 to 2:1 by weight.

10. The solid oral pharmaceutical composition according to claim 8, wherein the composition comprises one disintegrant which is croscarmellose sodium.

11. The solid oral pharmaceutical composition according to claim 10, wherein the ratio of croscarmellose sodium to the total weight of diluent is in the range of 1:1 to 1:4, preferably 1:2 to 1:3 and more preferably 1:2 to 1:2.5.

## Patentansprüche

1. Feste orale pharmazeutische Zusammensetzung, umfassend Tenofovir oder ein pharmazeutisch annehmbares Salz davon, Emtricitabin oder ein pharmazeutisch annehmbares Salz davon, und Lactosemonohydrat und mikrokristalline Cellulose als Verdünnungsmittel, wobei Lactosemonohydrat in einer Menge von 10-15 Gew.-% der Zusammensetzung vorliegt und die Zusammensetzung im Wesentlichen frei von Stärke ist.

2. Feste orale pharmazeutische Zusammensetzung nach Anspruch 1, wobei die Zusammensetzung Tenofovirdisoproxilfumarat und Emtricitabin umfasst.

3. Feste orale pharmazeutische Zusammensetzung nach Anspruch 2, wobei die Zusammensetzung Tenofovirdisoproxilfumarat in einer Menge von 5-60 Gew.-%, vorzugsweise 10-50 Gew.-% und noch bevorzugter 20-40 Gew.-% umfasst.

4. Feste orale pharmazeutische Zusammensetzung nach Anspruch 3, wobei die Zusammensetzung Emtricitabin in einer Menge von 5-60 Gew.-%, vorzugsweise 5-40 Gew.-% und besonders bevorzugt 10-30 Gew.-% umfasst.

5. Feste orale pharmazeutische Zusammensetzung nach Anspruch 4, wobei das Verhältnis von Tenofovirdisoproxilfumarat zu Emtricitabin im Bereich von 3:1 bis 0,5:1, vorzugsweise von 2:1 bis 1:1 und besonders bevorzugt von 1,5:1 liegt.

6. Feste orale pharmazeutische Zusammensetzung nach einem der vorhergehenden Ansprüche, wobei die Zusammensetzung in Form einer überzogenen Tablette, einer Dreischichttablette, einer Zweischichttablette, einer Mehrschichttablette, einer Schmelztablette, einer Minitablette, eines Pellets, eines Zuckerpellets, einer Buccaltablette, einer Sublingualtablette, einer Brausetablette, einer Tablette mit sofortiger Wirkstofffreisetzung, einer Tablette mit modifizierter Wirkstofffreisetzung, einer Filmtablette, einer sich im Magensaft auflösenden Tablette, einer Pille, einer Kapsel, eines oralen Granulats, eines Pulvers, eines beschichteten Beadsystems, in Form von Mikrokügelchen, einer kompressionsbeschichten Tablette, einer Punkttablette, eines Dragees, eines Beutels, eines oral verabreichbaren Films vorliegt.

7. Feste orale pharmazeutische Zusammensetzung nach Anspruch 6, wobei die Zusammensetzung in Form einer Filmtablette vorliegt.

8. Feste orale pharmazeutische Zusammensetzung nach einem der vorhergehenden Ansprüche, wobei die Zusammensetzung des Weiteren mindestens einen pharmazeutisch annehmbaren Hilfsstoff enthält, der ausgewählt ist aus Sprengmitteln, Gleitmitteln, Fließregulierungsmitteln oder Mischungen davon.

9. Feste orale pharmazeutische Zusammensetzung nach Anspruch 1, wobei das Gewichtsverhältnis von mikrokristalliner Cellulose zu Lactosemonohydrat im Bereich von 1:1 bis 3:1, vorzugsweise von 1:1 bis 2:1 und besonders bevorzugt von 1,5:1 bis 2:1 liegt.

10. Feste orale pharmazeutische Zusammensetzung nach Anspruch 8, wobei die Zusammensetzung ein Sprengmittel umfasst, bei dem es sich um Croscarmellose-Natrium handelt.

11. Feste orale pharmazeutische Zusammensetzung nach Anspruch 10, wobei das Verhältnis von Croscarmellose-Natrium zum Gesamtgewicht des Verdünnungsmittels im Bereich von 1:1 bis 1:4, vorzugsweise von 1:2 bis 1:3 und besonders bevorzugt von 1:2 bis 1:2,5 liegt.

## Revendications

1. Composition pharmaceutique orale solide comprenant du ténofovir ou un sel pharmaceutiquement acceptable de celui-ci, de l'emtricitabine ou un sel pharmaceutiquement acceptable de celle-ci et du lactose monohydraté et de la cellulose microcristalline en tant que diluants, dans laquelle le lactose monohydraté est dans une quantité de 10 à 15 % en poids de la composition et la composition est sensiblement exempte d'amidon.

2. Composition pharmaceutique orale solide selon la revendication 1, dans laquelle la composition comprend du fumarate de ténofovir disoproxil et de l'emtricitabine.

3. Composition pharmaceutique orale solide selon la revendication 2, dans laquelle la composition comprend du fumarate de ténofovir disoproxil dans une quantité de 5-60%, de préférence de 10-50% et de façon davantage préférée de 20-40% en poids.

4. Composition pharmaceutique orale solide selon la revendication 3, dans laquelle la composition comprend de l'emtricitabine dans une quantité de 5-60%, de préférence de 5-40% et de façon davantage préférée de 10-30% en poids.

5. Composition pharmaceutique orale solide selon la revendication 4, dans laquelle le rapport du fumarate de ténofovir disoproxil à l'emtricitabine est dans la plage de 3:1 à 0,5:1, de préférence de 2:1 à 1:1 et de façon davantage préférée ce rapport est de 1,5:1.

6. Composition pharmaceutique orale solide selon l'une quelconque des revendications précédentes, dans laquelle la composition se présente sous la forme d'un comprimé enrobé, d'un comprimé tricouches, d'un comprimé bicouches, d'un comprimé multicouches, d'un comprimé à délitement oral, d'un mini-comprimé, d'une pastille, d'une pastille de sucre, d'un comprimé buccal, d'un comprimé sublingual, d'un comprimé effervescent, d'un comprimé à libération immédiate, d'un comprimé à libération modifiée, d'un comprimé pelliculé, d'un comprimé à délitement gastrique, d'une pilule, d'une capsule, d'un granulé oral, d'une poudre, d'un système de billes enrobées, d'une microsphère, d'un comprimé dans un comprimé, d'un comprimé incrusté, d'une dragée, d'un sachet, d'un film administrable par voie orale.

7. Composition pharmaceutique orale solide selon la revendication 6, dans laquelle la composition se présente sous la forme d'un comprimé pelliculé.

8. Composition pharmaceutique orale solide selon l'une quelconque des revendications précédentes, dans laquelle la composition comprend en outre au moins un excipient pharmaceutiquement acceptable choisi parmi les agents délitants, les lubrifiants, les agents de glissement ou leurs mélanges.

9. Composition pharmaceutique orale solide selon la revendication 1, dans laquelle le rapport de la cellulose microcristalline au lactose monohydraté est dans la plage de 1:1 à 3:1, de préférence de 1:1 à 2:1 et de façon davantage préférée de 1,5:1 à 2:1 en poids.

10. Composition pharmaceutique orale solide selon la revendication 8, dans laquelle la composition comprend un agent délitant qui est la croscarmellose sodique.

11. Composition pharmaceutique orale solide selon la revendication 10, dans laquelle le rapport de la croscarmellose sodique au poids total du diluant est dans la plage de 1:1 à 1:4, de préférence de 1:2 à 1:3 et de façon davantage préférée de 1:2 à 1:2,5.
